(19) Europäisches Patentamt

European Patent Office

Office européen des brevets

(11) Veröffentlichungsnummer: **0 266 378 B1**

(12) **EUROPÄISCHE PATENTSCHRIFT**

(45) Veröffentlichungstag der Patentschrift:
04.09.91 Patentblatt 91/36

(21) Anmeldenummer: 87902418.0

(22) Anmeldetag: 06.05.87

(86) Internationale Anmeldenummer:
PCT/DE87/00197

(87) Internationale Veröffentlichungsnummer:
WO 87/06818 19.11.87 Gazette 87/25

(51) Int. Cl.⁵: **A61F 2/52**

(54) **BRUSTPROTHESE.**

(30) Priorität: 09.05.86 DE 3615726

(43) Veröffentlichungstag der Anmeldung:
11.05.88 Patentblatt 88/19

(45) Bekanntmachung des Hinweises auf die
Patenterteilung:
04.09.91 Patentblatt 91/36

(84) Benannte Vertragsstaaten:
AT BE CH DE FR GB IT LI LU NL SE

(56) Entgegenhaltungen:
EP-A- 0 178 483
WO-A-82/00583

(73) Patentinhaber: Schütt & Gründei
Orthopädietechnik GmbH
Grapengiesserstrasse 34
W-2400 Lübeck (DE)

(72) Erfinder: GRUNDEI, Hans
Gärtnergasse 4
W-2400 Lübeck (DE)

(74) Vertreter: Wilcken, Thomas, Dipl.-Ing. et al
Musterbahn 1
W-2400 Lübeck (DE)

## Beschreibung

Die Erfindung betrifft eine Brustprothese, bestehend aus einer elastisch verformbaren und zumindest teilweise mit einer träge fließenden Masse gefüllten Hülle, deren äußere Hüllenwand der Form der natürlichen Brustform nachgebildet ist und deren gegen den Brustkorb gerichtete innere Hüllenwand im wesentlichen eine konkav gekrümmte Form hat, während zumindest ein durch den Prothesenkörper verlaufender Kanal vorgesehen ist, der Öffnungen an den erwähnten inneren und äußeren Hüllwänden verbindet.

Aus der DE-OS 34 40 960 sind Brustprothesen bekannt, die eine Hülle aufweisen, in der eine träge fließende Masse sowie ein elastisch deformierbarer poröser Formkörper vorgesehen sind. Der elastische verformbare, poröse Formkörper der z.B. aus natürlichem oder künstlichem Schaum gebildet sein kann, hat im wesentlichen die Aufgabe, das Gewicht der Brustprothese zu reduzieren, während die träge fließende Masse im wesentlichen die Aufgabe hat, in Verbindung mit der Hülle die Form und Festigkeit einer natürlichen Brust zu imitieren, insbesondere die durch Körperbewegungen hervorgerufene natürliche Schwingbewegung der Brust sicherzustellen. Selbst diese schon gewichtsreduzierte Brustprothese weist, insbesondere in höheren Größen, noch ein solches - vom Büstenhalter aufzunehmendes - Gewicht auf, daß es im Bereich der Büstenhalterträger zu Einschnürungen an den Schultern der Trägerin kommen kann.

Im übrigen ist der Sitz der Prothese am Brustkorb nicht sicher gewährleistet.

Aus der WO 82/00 583 ist eine Brustprothese der eingangs erwähnten Art bekannt. Die dort beschriebene Prothese ist ebenfalls verhältnismäßig schwer, da das Prothesengewicht etwa dem einer natürlichen Brust entspricht. Die Fixierung dieser Prothese erfolgt durch Klebbefestigung am Brustkorb, wobei das Hauptgewicht der Brust durch den Büstenhalten aufgenommen werden muß. Weiterhin weist diese Brustprothese Entlüftungsbohrungen im Trägerrand des Mitteilteiles auf, um zu verhindern, daß die zwischen Brustprothese und Brustkorb befindliche Luft schlagartig unter charakeristischer Geräuschentwicklung entweicht.

Wegen des verhältnismäßig hohen Gewichts dieser Prothese wird insbesondere ihre Befestigung durch Ankleben am Brustkorb als unangenehm empfunden. Häufig erfolgt im Bereich dieser Klebverbindung eine Reizung der Haut. Da die Prothese nur fixiert und nicht vollflächig befestigt ist, kann es im Bereich der Klebestellen zu einem schmerzhaften Ziehen innerhalb der Haut kommen. Im übrigen ist das Anlegen dieser Prothese recht umständlich, da das Klebmittel regelmäßig erneuert werden muß und in Verbindung mit zahlreichen Pflegekosmetika nicht anwendbar ist.

Hiervon ausgehend liegt der Erfindung die Aufgabe zugrunde, eine Brustprothese der genannten Art so auszubilden, daß diese auf einfache,unkomplizierte und für die Trägerin angenehme Weise am Bruskorb fixierbar ist und zumindest ein Teil des Prothesengewichtes von der Trägerin direkt aufgenommen werden kann.

Diese Aufgabe wird erfindungsgemäß dadurch gelöst, daß innerhalb des Kanals ein Ventil angeordnet ist, das eine Luftströmung nur von innen nach außen zuläßt.

Durch diese Lösung braucht die Trägerin die Prothese nur einmal oder einige Male gegen die Brust zu drücken und dann wieder loszulassen, so daß dann zwischen der Prothese und der Brust ein Unterdruck entsteht, durch den sich vor allem der Umfangsrand der Prothese abdichtend gegen den Brustkorb anlegt, womit eine feste Lage der Prothese erreicht ist und womit das Prothesengewicht, zumindest ein Teil desselben vom Brustkorb direkt aufgenommen wird.

Der vorerwähnte Kanal ist nach einer weiteren Ausbildung durch einen Schlauch gebildet, der mit dem in der Hülle vorgesehenen Ventil verbunden ist. Dabei liegt das Ventil im Brustwarzenbereich der Hülle, der entsprechend verdickt ist und z.B. in der Hülle einvulkanisiert ist.

Vorteilhaft ist, daß die Prothese auf der der Brust zugekehrten Fläche in Nähe des Umfangsrandes mit einer umlaufenden Nut versehen ist, die mit einer am Brustkorb haftenden Dichtungsmasse ausgefüllt ist.

Um die Entstehung eines Unterdruckes im Bereich zwischen der Prothese und der Brustkorbfläche zu gewährleisten, ist die dem Brustkorb zugekehrte Wandung der Prothesenhülle formstabil ausgebildet und räumlich so gekrümmt, daß sich zwischen der Prothese und dem Brustkorb ein Hohlraum bildet. Nach einmaligem oder mehrmaligem Zusammendrücken der Prothese wird die Luft aus dem Raum zwischen der Prothese und dem Brustkorb nach jedesmaligem Loslassen der Prothese über das Rückschlagventil abströmen und dadurch wird in diesem Raum durch die Rückkehr der Prothese in die Ausgangsform ein Unterdruck erzeugt, durch den die Prothese an ihrer angesetzten Stelle festgelegt wird und wobei das Gewicht vom Brustkorb zumindest teilweise aufgenommen wird, so daß dann ein Büstenhalter praktisch nur noch ein geringes Gewicht aufzunehmen hat und eine zusätzliche Haltesicherung bildet.

Die Erfindung wird nachstehend anhand der Zeichnung erläutert, in der ein Ausführungsbeispiel einer Brustprothese dargestellt ist. Es zeigen:

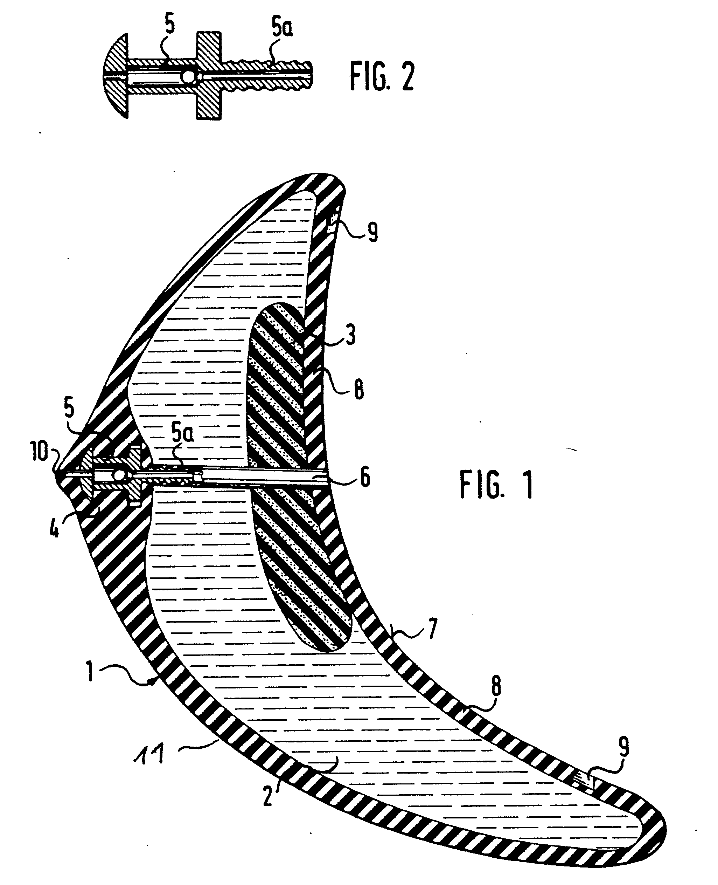

Figur 1 einen waagerechten Schnitt durch eine Brustprothese nach der Erfindung,

Figur 2 einen Schnitt durch das im Achsenbereich der Prothese eingebaute Rückschlagventil in vergrößertem Maßstab.

Die Prothese als Ersatz für eine amputierte Brust besteht aus einer formstabilen, elastischen Hülle 1, die der natürlichen Brust nachgeformt ist. Diese Hülle 1 besteht vorteilhaft aus einem vulkanisierten Silikonkautschuk und ist mit einer träge fließenden Masse 2, z.B. einem teilvulkanisierten Silikonkautschukgel, gefüllt. Der Füllraum 2 kann teilweise zur Gewichtsverminderung von einem natürlichen oder künstlichen Schaumkörper 3 eingenommen werden.

Die äußere Hüllenwand 11 ist im Warzenbereich 4 verdickt, und in diese Verdickung ist ein Rückschlagventil 5 einvulkanisiert. Dieses in Figur 3 vergrößert dargestellte Rückschlagventil 5 ist mit einem in den Hüllenraum 2 frei hineinragenden Stutzen 5a versehen, auf den das Ende eines Schlauches 6 aufgeschoben ist, der quer durch die Prothese verläuft und der in der gegen die Brust gerichteten Flächen 7 der inneren Hüllenwand 8 endet. Das der Brust zugekehrte Ende des Schlauches 6 kann trompetenförmig erweitert sein oder in einem offenzelligen Ansatz, z.B. einem Schaumkörper, enden. Der Ansatz, der auch starr sein kann, ist lösbar mit dem Schlauch und/order der inneren Hüllenwand 8 verbunden.

Die der Brust zugekehrte innere Hüllenwand 8 ist in Nähe des Umfangsrandes mit einer Nut, z.B. einer hinterschnittenen Nut versehen, in die eine am Brustkorb dichtend haftende Masse 9, z.B. ein entsprechend eingestelltes Silikonkautschukgel 9, eingefüllt ist. Die Außenfläche der inneren Hüllenwand 8 ist formstabil so gekrümmt, daß sich beim Ansetzen der Prothese an die Brust zwischen der inneren Hüllenwand 8 und der Brustfläche ein Hohlraum bildet. Damit dieser Hohlraum nach Ansetzen der Prothese an die Brustfläche über den Schlauch 6 und das Rückschlagventil 5 in die Außenluft entlüftet werden kann, ist die Wandung 4 im Bereich des Ventils 5 mit einem Durchstich 10 versehen.

Die Brustprothese wird so an den Brustkorb angelegt, daß die Dichtungsmasse 9 im Randbereich der inneren Hüllenwand 8 an den Brustkorb anliegt, so daß die Prothese dadurch am Brustkorb haftend gehalten wird. Sodann wird die Prothese als Ganzes durch Handdruck gegen den Brustkorb gedrückt, so daß dadurch die Luft aus dem Hohlraum zwischen der inneren Hüllenwand 8 und dem Brustkorb über den Schlauch 6, das Rückschlagventil 5 und den Durchstich 10 wenigstens teilweise nach außen entweicht. Läßt man die Prothese los, so versucht die verformte Prothese durch die Elastizität ihrer Hülle ihre Ausgangslage wieder einzunehmen, und dadurch wird der Raum zwischen der inneren Hüllenwand 8 und dem Brustkorb vergrößert, so daß in diesem Raum ein Unterdruck entsteht, d.h. die Kugel des Rückschlagventils 5 legt sich auf ihren Sitz, so daß der Unterdruck im vorerwähnten Raum aufrechterhalten wird. Dieser Vorgang kann mehrere Male wiederholt werden, bis der gewollte Unterdruck im Raum zwischen der inneren Hüllenwand 8 und dem Brustkorb erreicht ist. Damit sitzt die Prothese fest an der richtigen Stelle und das Gewicht der Prothese wird damit zumindest teilweise vom Brustkorb selbst aufgenommen, so daß der zur Anwendung kommende Büstenhalter und dessen Träger entlastet werden.

## Patentansprüche

1. Brustprothese, bestehend aus einer elastisch verformbaren und zumindest teilweise mit einer träge fließenden Masse gefüllten Hülle, deren äußere Hüllenwand (11) der form der natürlichen Brustform nachgebildet ist und deren gegen den Brustkorb gerichtete innere Hüllenwand (8) im wesentlichen eine konkav gekrümmte Form hat, während zumindest ein durch den Prothesenkörper verlaufender Kanal (6) vorgesehen ist, der Öffnungen an den erwähnten inneren und äußeren Hüllwänden (8 und 11) verbindet, dadurch gekennzeichnet, daß im Kanal ein Ventil (5) angeordnet ist, das eine Luftströmung nur von innen nach außen zuläßt.

2. Brustprothese nach Anspruch 1, dadurch gekennzeichnet, daß der Kanal (6) durch einen flexiblen Schlauch (6) gebildet ist, der mit dem in der Hülle (1) steckenden Ventil (5) verbunden ist.

3. Brustprothese nach Anspruch 2, dadurch gekennzeichnet, daß in dem die Brustwarze nachbildenden Bereich der Hülle (1) eine Materialverdickung (4) vorgesehen ist, in der sich das gegen axiale Verschiebung gesicherte Ventil (5) befindet.

4. Brustprothese nach einem der Ansprüche 1 bis 3, dadurch gekennzeichnet, daß der Kanal (6) am körperseitigen Ende in einen flexiblen, sich trompetenförmig erweiternden Ansatz übergeht.

5. Brustprothese nach einem der Ansprüche 1 bis 3, dadurch gekennzeichnet, daß der Kanal (6) am körperseitigen Ende in einem offenzelligen, an der genannten inneren Hüllenwand (8) liegenden Ansatz übergeht.

6. Brustprothese nach einem der Ansprüche 4 und 5, dadurch gekennzeichnet, daß der Ansatz lösbar mit dem Kanal (6) und/order der inneren Hüllenwand (8) verbunden ist.

7. Brustprothese nach einem der Ansprüche 1 bis 6, dadurch gekennzeichnet, daß in Nähe des äußeren Randbereiches der inneren Hüllenwand (8) eine umlaufende Dichtung (9) vorgesehen ist.

8. Brustprothese nach Anspruch 7, dadurch gekennzeichnet, daß die Dichtung (9) in einer Ausnehmung der inneren Hüllenwand (8) eingelassen ist und aus einem haftenden Material besteht.

9. Brustprothese nach einem der Ansprüche 1 bis 8, dadurch gekennzeichnet, daß die dem Brustkorb zugekehrte innere Hüllenwand (8) im unbelasteten Zustand formstabil und räumlich gekrümmt ist, derart, daß sich beim anfänglichen Anlegen der Prothese ein Abstand und freier Raum zwischen der inneren Hüllenwand (8) und dem Brustkorb bildet.

10. Brustprothese nach einem der vorhergehenden Ansprüche, dadurch gekennzeichnet, daß die Hülle aus einem vulkanisierten Silikonkautschuk besteht und mindestens teilweise mit einem nur teilvulkanisierten Silikonkautschuk gefüllt ist, wobei das Rückschlagventil (5) in der Wandungsverdickung (4) der Hülle (1) im Brustwarzenbereich einvulkanisiert ist und einen Stutzen aufweist, über den es mit dem Schlauch (6) verbunden ist.

## Claims

1. Breast prosthesis, consisting of an elastically deformable shell, which is at least partially filled with a slowly flowing substance, the outer shell wall (11) of which imitates the natural breast shape and the inner shell wall (8) of which, facing the rib cage, has an essentially concave shape, while at least one duct (6) is provided which extends through the prosthesis body and connects openings at the above-mentioned inner and outer shell walls (8 and 11), characterised in that a valve (5), which only permits air to flow from the inside outwards, is arranged in the duct.

2. Breast prosthesis according to claim 1, characterised in that the duct (6) is formed by a flexible tube (6), which is connected to the valve (5) fitted in the shell (1).

3. Breast prosthesis according to claim 2, characterised in that a material thickening (4) is provided in the area of the shell (1) which imitates the nipple, in which thickening the valve (5), which is secured against axial displacement, is disposed.

4. Breast prosthesis according to one of claims 1 to 3, characterised in that the duct (6) passes into a flexible attachment, which widens like a trumpet, at the end which is on the body side.

5. Breast prosthesis according to one of claims 1 to 3, characterised in that the duct (6) passes into an open-cell attachment, which lies against the said inner shell wall (8), at the end which is on the body side.

6. Breast prosthesis according to one of claims 4 and 5, characterised in that the attachment is connected to the duct (6) and/or the inner shell wall (8) in a detachable manner.

7. Breast prosthesis according to one of claims 1 to 6, characterised in that a continuous seal (9) is provided in the vicinity of the outer edge area of the inner shell wall (8).

8. Breast prosthesis according to claim 7, characterised in that the seal (9) is set in a recess in the inner shell wall (8) and consists of an adhesive material.

9. Breast prosthesis according to one of claims 1 to 8, characterised in that the inner shell wall (8), which faces the rib cage, is curved in an inherently and three-dimensionally stable manner when unstressed such that a clearance and free space is formed between the inner shell wall (8) and the rib cage when the prosthesis is initially applied.

10. Breast prosthesis according to one of the preceding claims, characterised in that the shell consists of a vulcanized silicone rubber and is at least partially filled with a silicone rubber which is only partially vulcanized, the non-return valve (5) being vulcanized into the wall thickening (4) of the shell (1) in the nipple area and comprising a connection piece via which it is connected to the tube (6).

## Revendications

1. Prothèse du sein, composée d'une enveloppe élastiquement déformable et au moins partiellement remplie d'une masse à écoulement retardé, dont la paroi d'enveloppe extérieure (11) reproduit la forme du sein naturel et dont la paroi intérieure d'enveloppe (8) tournée vers la cage thoracique présente sensiblement une forme incurvée de façon concave, tandis qu'il est prévu un canal (6), traversant le corps de prothèse, qui relie des orifices sur les parois d'enveloppe (8 et 11) intérieure et extérieure mentionnées, caractérisée en ce qu'une valve (5), qui ne permet l'écoulement de l'air que de l'intérieur vers l'extérieur, est disposée dans le canal.

2. Prothèse de sein selon la revendication 1, caractérisée en ce que le canal (6) est constitué par un tube flexible (6) qui est relié à la valve (5) qui est enfoncée dans l'enveloppe (1).

3. Prothèse de sein selon la revendication 2, caractérisée en ce qu'il est prévu, dans la zone de l'enveloppe (1) qui représente le mamelon, un épaississement de matière (4) dans lequel se trouve la valve (5) qui ne peut être déplacée dans le sens axial.

4. Prothèse du sein selon l'une des revendications 1 à 3, caractérisée en ce que le canal (6) se raccorde sur l'extrémité côté corps dans un appendice flexible, qui s'évase en forme de trompette.

5. Prothèse du sein selon l'une des revendications 1 à 3, caractérisée en ce que le canal (6) se raccorde sur l'extrémité côté corps dans un appendice à cellules ouvertes, situé sur la paroi d'enveloppe intérieure mentionnée (8).

6. Prothèse du sein selon l'une des revendications 4 ou 5, caractérisée en ce que l'appendice est relié au canal (6) et/ou à la paroi d'enveloppe intérieure (8) de manière détachable.

7. Prothèse du sein selon l'une des revendications 1 à 6, caractérisée en ce qu'un joint d'étanchéité périphérique (9) est prévu au voisinage de la zone de bord extérieur de la paroi d'enveloppe intérieure (8).

8. Prothèse du sein selon la revendication 7, caractérisée en ce que le joint d'étanchéité (9) est introduit dans un évidement de la paroi d'enveloppe intérieure (8) et se compose d'une matière adhésive.

9. Prothèse du sein selon l'une des revendications 1 à 8, caractérisée en ce que la partie d'enveloppe intérieure (8) tournée vers la cage thoracique est de forme stable et incurvée dans l'espace dans l'état non chargé de telle manière que, lorsque la prothèse est placée à l'origine, il existe une distance et un espace libre entre la paroi intérieure d'enveloppe (8) et la cage thoracique.

10. Prothèse du sein selon l'une des revendications précédentes, caractérisée en ce que l'enveloppe est en caoutchouc de silicone vulcanisé et est remplie au moins partiellement d'un caoutchouc de silicone qui n'est que partiellement vulcanisé, le clapet anti-retour (5) étant vulcanisé dans l'épaississement (4) de paroi de l'enveloppe (1) dans la zone de la cage thoracique et présentant un piquage grâce auquel il est relié au tube (6).

FIG. 2

FIG. 1